(19) Europäisches Patentamt
European Patent Office
Office européen des brevets

(11) Veröffentlichungsnummer: **0 006 176**
**B1**

# (12) EUROPÄISCHE PATENTSCHRIFT

(45) Veröffentlichungstag der Patentschrift: **06.01.82**

(21) Anmeldenummer: **79101763.5**

(22) Anmeldetag: **05.06.79**

(51) Int. Cl.³: **C 07 D 498/04,** C 07 D 513/04, D 06 L 3/12, C 11 D 3/42, C 08 J 3/20

## (54) Neue Stilbenverbindungen, Verfahren zu deren Herstellung und deren Verwendung als optische Aufheller.

(30) Priorität: **09.06.78 CH 6347/78**

(43) Veröffentlichungstag der Anmeldung: **09.01.80 Patentblatt 80/1**

(45) Bekanntmachung des Hinweises auf die Patenterteilung: **06.01.82 Patentblatt 82/1**

(84) Benannte Vertragsstaaten: **CH DE FR GB IT**

(56) Entgegenhaltungen: **FR-A-2 046 545**

(73) Patentinhaber: **HOECHST AKTIENGESELLSCHAFT, Postfach 80 03 20, D-6230 Frankfurt/Main 80 (DE)**

(72) Erfinder: **Günther, Dieter, Dr., Nachtigallenweg 1A, D-6233 Kelkheim (Taunus) (DE)**
Erfinder: **Eckes, Helmut, Dr., Feldbergblick 5, D-6239 Eppstein/Taunus (DE)**
Erfinder: **Erckel, Rüdiger, Dr., Staufenstrasse 16, D-6239 Eppstein/Taunus (DE)**
Erfinder: **Rösch, Günter, Hohlweg 17, D-6232 Bad Soden am Taunus (DE)**

**EP 0 006 176 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Neue Stilbenverbindungen, Verfahren zu deren Herstellung und deren Verwendung als optische Aufheller

Aus der französischen Patentschrift 2 046 545 sind bereits optische Aufheller aus der Reihe der Stilbenverbindungen bekannt, die symmetrisch durch zwei gleiche Pyridoxazolyl-Gruppen substituiert sind. Es wurde nun überraschend gefunden, daß man den Weißgrad derartiger Verbindungen noch erhöhen kann, wenn man eine der beiden Pyridoxazolyl-Gruppen durch eine Oxadiazolyl-Gruppe ersetzt, so daß die Verbindung symmetrisch wird.

Gegenstand der Erfindung sind somit Verbindungen der allgemeinen Formel

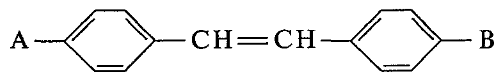

wobei A eine Gruppe der Formeln

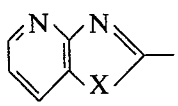 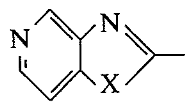 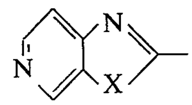

oder

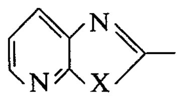

bedeutet, deren Pyridinring durch Fluor- oder Chloratome oder $C_1-C_4$-Alkylgruppen substituiert sein kann und B eine Gruppe der Formeln

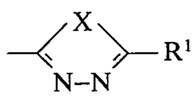 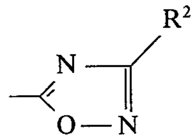 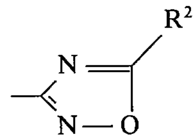

CN, $COOR^3$ oder 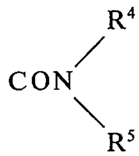

ist, wobei $R^1$ einen Phenylring, der durch 1 oder 2 Chloratome, ein oder zwei $C_1-C_4$-Alkyl- oder $C_1-C_4$-Alkoxy-$C_1-C_4$-alkylgruppen substituiert sein kann, $R^2$, $R^3$, $R^4$ und $R^5$ die gleiche Bedeutung wie $R^1$ haben und zusätzlich jeweils eine $C_1-C_{18}$-Alkylgruppe, eine Benzyl-, $C_1-C_6$-Chloralkyl-, $C_1-C_4$-Alkoxy-$C_1-C_4$-alkyl- oder $C_1-C_4$-Hydroxyalkylgruppe oder eine Gruppe der Formel $-(CH_2CH_2O)_n-R'$ mit $R'$=Wasserstoff oder $C_1-C_4$-Alkyl und $n = 1$ oder 2 bedeuten, $R^3$, $R^4$ und $R^5$ zusätzlich auch ein Wasserstoffatom bedeuten und $R^4$ und $R^5$ zusammen mit dem Stickstoffatom auch einen Morpholin-, Piperidin- oder Piperazinring bedeuten und X ein Sauerstoff- oder Schwefelatom bedeutet.

Von allen beanspruchten Verbindungen sind jeweils diejenigen bevorzugt, wo sowohl im Heterocyclus unter A als auch unter B X ein Sauerstoffatom bedeutet.

Bevorzugt sind Verbindungen der beiden allgemeinen Formeln 2 und 3

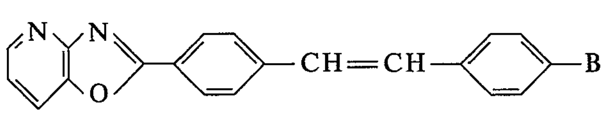 (2)

oder

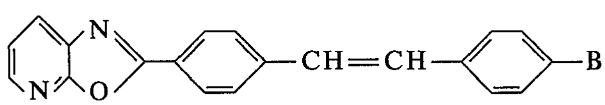 (3)

mit der oben angegebenen Bedeutung für B, wobei $R^1$ einen Phenylring, der durch ein oder zwei Chloratome, eine oder zwei $C_1-C_4$-Alkyl- oder $C_1-C_4$-Alkoxy-$C_1-C_4$-alkyl-gruppen substituiert sein

kann, $R^2$, $R^3$, $R^4$ und $R^5$ die gleiche Bedeutung wie $R^1$ haben und zusätzlich jeweils eine $C_1-C_6$-Alkyl-, $C_1-C_6$-Chloralkyl-, $C_1-C_4$-Alkoxy-$C_1-C_4$-alkyl-, $C_1-C_4$-Hydroxyalkyl-, Benzylgruppe oder eine Gruppe der Formel $-(CH_2CH_2O)_n-R'$ mit $n = 1$ oder 2 und $R'$ = Wasserstoff oder $C_1-C_4$-alkyl bedeuten und $R^3$, $R^4$ und $R^5$ zusätzlich auch ein Wasserstoffatom bedeuten können.

Besonders bevorzugt sind Verbindungen der beiden Formeln 2 und 3 mit der oben angegebenen Bedeutung für B, wobei $R^1$ Phenyl, mono- oder di-$C_1-C_2$-Alkylphenyl, $C_1-C_2$-Alkoxyphenyl, Chlorphenyl oder Dichlorphenyl, $R^2$, $R^3$, $R^4$ und $R^5$ die gleiche Bedeutung wie $R^1$ haben und zusätzlich jeweils $C_1-C_5$-Alkyl, $C_1-C_2$-Chloralkyl, $C_1-C_2$-Hydroxylalkyl, $C_1-C_2$-Alkoxy-$C_1-C_2$-alkyl oder Benzyl und $R^3$, $R^4$ und $R^5$ zusätzlich auch ein Wasserstoffatom bedeuten können.

Gegenstand der Erfindung ist weiterhin ein Verfahren zur Herstellung der Verbindungen der Formeln 1 bzw. 2 und 3, wobei man

a) wenn B eine Gruppe der Formel $-COOR^3$ oder

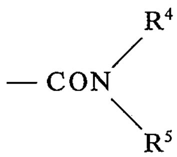

bedeutet, eine Carbonsäure der allgemeinen Formel

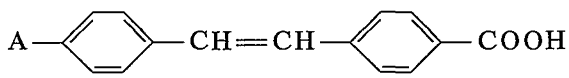

oder ein funktionelles Derivat dieser Säure mit einer Verbindung der allgemeinen Formel

$HOR^3$ oder 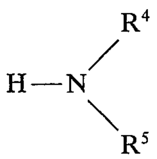

umsetzt, oder

b) wenn B eine Cyanogruppe bedeutet, aus einer Verbindung der allgemeinen Formel

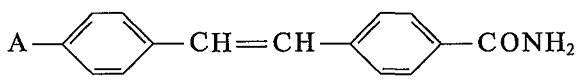

Wasser abspaltet, oder

c) wenn B eine Gruppe der Formel

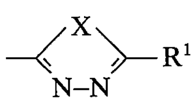

bedeutet, entweder eine Säure der Formel

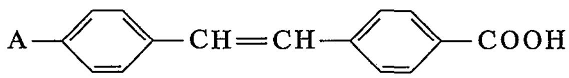

oder ein funktionelles Derivat dieser Säure mit einem Carbonsäurehydrazid der Formel

$H_2NNHCOR^1$

oder einer Säure der Formel

$R^1-COOH$

oder ein funktionelles Derivat dieser Säure mit einer Verbindung der Formel

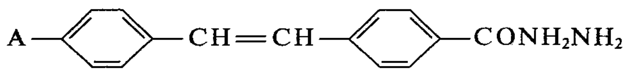

umsetzt und anschließend mit einem wasserabspaltenden Mittel cyclisiert, wobei, wenn X ein Schwefelatom bedeutet, die Cyclisierung mit $P_2S_5$ erfolgt, oder

d) wenn B eine Gruppe der Formel

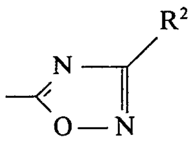

bedeutet, eine Säure der Formel

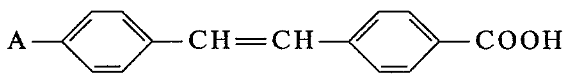

oder ein funktionelles Derivat dieser Säure mit einem Amidoxim der Formel

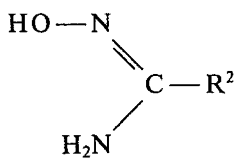

umsetzt und mit einem wasserabspaltenden Mittel cyclisiert, oder

e) wenn B eine Gruppe der Formel

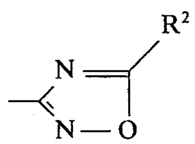

bedeutet, eine Verbindung der Formel

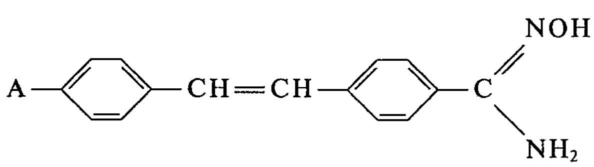

mit einem Carbonsäurechlorid der Formel

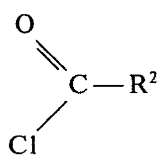

umsetzt und mit einem wasserabspaltenden Mittel cyclisiert, oder

f) eine Verbindung der Formel

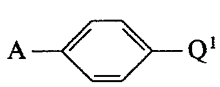

mit einer Verbindung der Formel

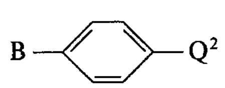

umsetzt, wobei eines der Symbole $Q^1$ oder $Q^2$ eine Aldehydgruppe und das andere Symbol

eine Gruppe der Formeln

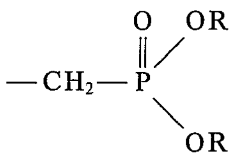

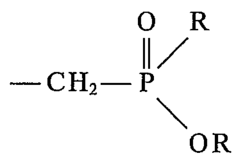

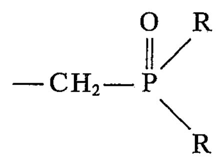

oder

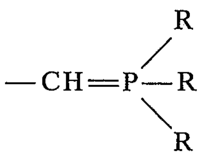

bedeutet, wobei R einen gegebenenfalls substituierten Alkylrest mit vorzugsweise 1–6 C-Atomen, einen Arylrest, vorzugsweise Phenyl, einen Cycloalkylrest oder einen Aralkylrest, vorzugsweise Benzyl, darstellt.

Das gemeinsame Ausgangsprodukt für die Verfahren a) bis e) ist die Verbindung der Formel

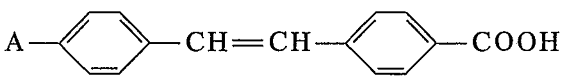

Je nach der Bedeutung von A läßt sich diese Säure nach an sich bekannten Verfahren herstellen, wie in der folgenden Übersicht schematisch dargestellt.

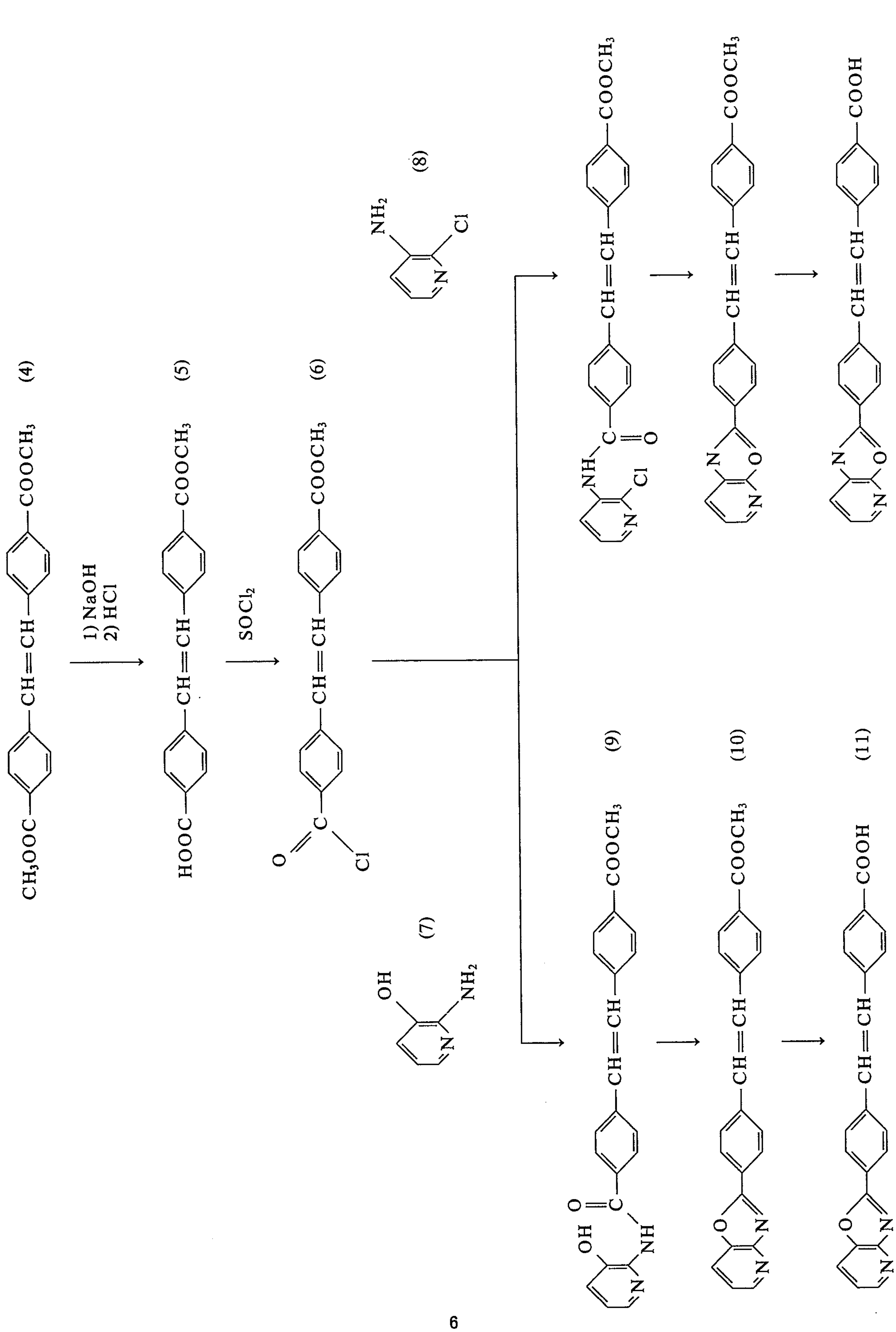


(4) (5) (6) (7) (8) (9) (10) (11) (12) (13) (14)

Die Umsetzung von Verbindungen der Formel (6) mit Pyridinderivaten der Formel (7) zum Produkt (10) kann mit oder ohne Zwischenisolierung von (9) durch Erhitzen auf höhere Temperaturen beispielsweise 120 bis 330°, vorteilhaft unter einem Inertgas, z. B. einem Stickstoffstrom, erfolgen, wobei die Reaktion gegebenenfalls in Gegenwart eines Katalysators durchgeführt wird. Geeignete Katalysatoren sind z. B. Borsäure, Zinkchlorid, p-Toluolsulfonsäure, ferner Polyphosphorsäuren einschließlich Pyrophosphorsäure.

Arbeitet man mit Borsäure als Katalysator, so verwendet man diesen vorteilhaft in einer Menge von etwa 0,5 bis 5%, bezogen auf das Gesamtgewicht der Reaktionsmasse. Es können auch hochsiedende, polare organische Lösungsmittel, wie z. B. N-Methylpyrrolidon, Dimethylformamid und aliphatische, gegebenenfalls verätherte Oxyverbindungen, z. B. Dialkylcarbinole, Propylenglykol, Äthylenglykolmonomethyläther oder Diäthylenglykoldiäthyläther und hochsiedende Ester der Phthalsäure, wie z. B. Phthalsäuredibutylester mit verwendet werden.

Man kann diese Reaktion aber auch in zwei Stufen durchführen, indem man das Carbonsäurechlorid der Formel (6) mit dem Pyridinderivat der Formel (7) oder (8) in Gegenwart eines organischen Lösungsmittels wie beispielsweise Toluol, Xylolen, Chlorbenzol, Dichlorbenzol, Tetralin, Trichlorbenzol oder Nitrobenzol bei höheren Temperaturen kondensiert und dann die dabei erhaltenen N-Acylverbindungen (9) bzw. (12) bei höheren Temperaturen, gegebenenfalls in Gegenwart eines Katalysators in die Verbindungen (10) bzw. (13) überführt. Diese Arbeitsweise ist besonders bei dem Pyridinderivat (8) vorteilhaft. In diesem Fall wird die Cyclierung der erhaltenen N-Acylaminoverbindung (12) zu (13) so vorgenommen, wie sie z. B. in GB-PS 1 242 836 oder US-PS 3 873 531 beschrieben ist, in Gegenwart von Kupferacetat und metallischem Zinkpulver.

Von den Estern (10) und (13) gelangt man durch einfache Verseifungsreaktion zu den Verbindungen (11) und (14) (z. B. wie in DE-OS 1 294 917 angegeben).

Analog zu der oben beschriebenen Umsetzung mit der Verbindung (7) kann man auch die Verbindung der Formel

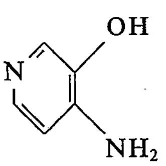

verwenden. In gleicher Weise kann man anstelle der Verbindung (8) die Verbindung der Formel

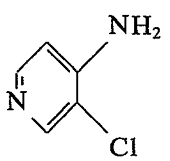

einsetzen.

Die auf diese Weise erhältlichen Carbonsäuren 11 bzw. 14 dienen direkt als Ausgangsverbindungen in den oben beschriebenen Verfahrensvarianten a), c) und b). Anstelle der freien Carbonsäuren kann man auch deren funktionelle Derivate, vorzugsweise die Säurechloride verwenden. Für das Verfahren b) werden die Carbonsäure 11 bzw. 14 auf üblichem Wege über das Carbonamid in das entsprechende Nitril überführt. Das als Ausgangsverbindung für die Verfahrensvariante e) dienende Amidoxim erhält man ebenfalls nach bekannten Verfahren aus diesen Nitrilen durch Addition von Hydroxylamin (DE-OS 2 709 924). Das Carbonsäurehydrazid für die Verfahrensvariante c) wird erhalten durch Umsetzung der Carbonsäuren 11 oder 14 oder deren Ester mit Hydrazin.

Die bei der Verfahrensvariante f) benötigten, Phosphoratome enthaltenden Ausgangsverbindungen werden erhalten, indem man nach bekannten Verfahren Halogenmethylverbindungen, vorzugsweise Chlormethyl- oder Brommethylverbindungen der Formeln

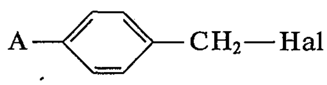 bzw. 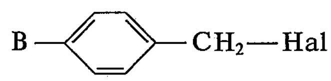

wobei A und B die obengenannten Bedeutungen haben und Hal vorzugsweise Chlor oder Brom bedeuten, mit Phosphorverbindungen der Formeln

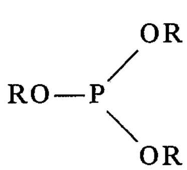 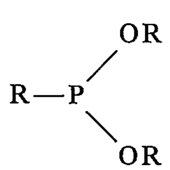 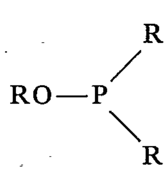 oder 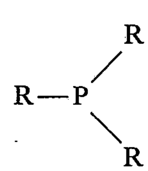

umsetzt, wobei R die oben angegebenen Bedeutungen hat. Die unmittelbar am Phosphor gebundenen Reste R bedeuten vorzugsweise Arylreste wie Phenyl, während die am Sauerstoff gebundenen Reste R vorzugsweise niedere Alkylgruppen bedeuten. Die Phosphoratome enthaltenden Ausgangsverbindungen für die Verfahrensvariante f) können auch erhalten werden, indem man die oben dargestellten Halogenmethylverbindungen mit U-pChlordiphenylphosphin und anschließend mit einem Alkohol der Formel R—OH umsetzt, wobei R Wasserstoff oder einen Rest R wie oben definiert, bedeuten. Die Umsetzung der ein Phosphoratom enthaltenden Ausgangsverbindungen mit den Verbindungen mit Aldehydfunktion gemäß Verfahrensvariante f) erfolgt vorteilhaft in indifferenten Lösungsmitteln.

Als Beispiele hierfür seien Kohlenwasserstoffe wie Toluol und Xylol oder Alkohole wie Methanol, Äthanol, Isopropanol, Butanol, Glykole, Glykoläther wie 2-Methoxyäthanol, Hexanole, Cyclohexanol und Cyclooctanol, ferner Äther wie Diisopropyläther, Tetrahydrofuran und Dioxan sowie Dimethylsulfoxid, Formamid und N-Methylpyrrolidon genannt. Besonders geeignet sind polare organische Lösungsmittel wie Dimethylformamid, N-Methylpyrrolidon und Dimethylsulfoxid. Auch in wäßriger Lösung lassen sich einige der Umsetzungen durchführen.

Die Temperatur, bei welcher die Umsetzung durchgeführt wird, kann in weiten Grenzen schwanken. Sie wird bestimmt

α) durch die Beständigkeit des verwendeten Lösungsmittels gegenüber den Reaktionsteilnehmern, insbesondere gegenüber den stark basischen Alkaliverbindungen,
β) durch die Reaktivität der Kondensationspartner und
γ) durch die Wirksamkeit der Kombination Lösungsmittel-Base als Kondensationsmittel.

In der Praxis kommen hiernach im allgemeinen Temperaturen zwischen etwa 10 und 100°C in Betracht, insbesondere, wenn Dimethylformamid oder Dimethylsulfoxid als Lösungsmittel verwendet werden. Der Temperatur-Vorzugsbereich liegt bei 20 bis 60°C. Es können jedoch unter Umständen auch höhere Temperaturen angewandt werden, wenn dies aus Gründen der Zeitersparnis erwünscht ist, oder ein weniger aktives, dafür aber billigeres Kondensationsmittel eingesetzt werden soll.

Grundsätzlich sind somit auch Reaktionstemperaturen im Intervall von 10 bis 180°C möglich.

Als stark basische Alkaliverbindungen kommen vor allem die Hydroxide, Amide und Alkoholate (vorzugsweise solche primärer, 1 bis 4 Kohlenstoffatome enthaltender Alkohole) der Alkalimetalle in Betracht, wobei aus ökonomischen Gründen solche des Lithiums, Natriums und Kaliums von vorwiegendem Interesse sind. Es können jedoch grundsätzlich und in besonderen Fällen auch Alkalisulfide und -carbonate, Arylalkaliverbindungen wie z. B. Phenyl-lithium, oder stark basische Amine (einschließlich Ammoniumbasen, z. B. Trialkylammoniumhydroxide) mit Erfolg verwendet werden.

Die vorstehend definierten neuen Verbindungen zeigen in gelöstem oder feinverteiltem Zustand eine mehr oder weniger ausgeprägte Fluoreszenz. Sie eignen sich deshalb zum optischen Aufhellen der verschiedensten synthetischen, halbsynthetischen oder natürlichen organischen Materialien oder Substanzen, welche solche organischen Materialien enthalten.

Hierfür seien beispielsweise, ohne daß durch die nachfolgende Übersicht irgendeine Beschränkung hierauf ausgedrückt werden soll, die folgenden Gruppen von organischen Materialien, soweit eine optische Aufhellung derselben in Betracht kommt, genannt:

I. Synthetische organische hochmolekulare Materialien:
  a) Polymerisationsprodukte auf Basis mindestens eine polymerisierbare Kohlenstoff-Kohlenstoff-Doppelbindung enthaltender organischer Verbindungen, d. h. deren Homo- oder Copolymerisate sowie deren Nachbehandlungsprodukte wie beispielsweise Vernetzungs-, Pfropfungs- oder Abbauprodukte, Polymerisat-Verschnitte oder durch Modifizierung reaktionsfähiger Gruppen erhaltene Produkte, beispielsweise Polymerisate auf Basis von α,β-ungesättigten Carbonsäuren oder Derivaten solcher Carbonsäuren, insbesondere von Acrylverbindungen (wie z. B. Acrylestern, Acrylsäure, Acrylnitril, Acrylamiden und deren Derivaten oder deren Methacryl-Analoga), von Olefin-Kohlenwasserstoffen (wie z. B. Äthylen, Propylen, Styrole oder Diene, ferner sogenannte ABS-Polymerisate), Polymerisate auf Basis von Vinyl- und Vinyliden-Verbindungen (wie z. B. Vinylchlorid, Vinylalkohol, Vinylidenchlorid),
  b) Polymerisationsprodukte, die durch Ringöffnung erhältlich sind, z. B. Polyamide vom Polycaprolactam-Typ, ferner Polymere, die sowohl über Polyaddition als auch Polykondensation erhältlich sind, wie Polyäther oder Polyacetale,
  c) Polykondensationsprodukte oder Vorkondensate auf Basis bi- oder polyfunktioneller Verbindungen mit kondensationsfähigen Gruppen, deren Homo- und Mischkondensationsprodukte sowie Produkte der Nachbehandlung, wie beispielsweise Polyester, insbesondere gesättigte (z. B. Äthylenglykolterephthalsäure-Polyester) oder ungesättigte (z. B. Maleinsäure-Dialkohol-Polykondensate sowie deren Vernetzungsprodukte mit anpolymerisierbaren Vinylmonomeren), unverzweigte sowie verzweigte (auch auf Basis höherwertiger Alkohole, wie z. B. Alkydharze) Polyester, Polyamide (z. B. Hexamethylendiamin-adipat), Maleinharze,

Melaminharze, deren Vorkondensate und Analoga, Polycarbonate, Silikone,
d) Polyadditionsprodukte wie Polyurethane (vernetzt und unvernetzt), Epoxidharze.

II. Halbsynthetische organische Materialien, z. B. Celluloseester verschiedener Veresterungsgrade (sogenanntes 2$^1/_2$-Acetat, Triacetat) oder Celluloseäther, regenerierte Cellulose (Viskose, Kupferammoniak-Cellulose) oder deren Nachbehandlungsprodukte, Casein-Kunststoffe.

III. Natürliche organische Materialien animalischen oder vegetabilischen Ursprungs, beispielsweise auf Basis von Cellulose oder Proteinen wie Baumwolle, Wolle, Leinen, Seiden, natürliche Lackharze, Stärke, Casein.

Die optisch aufzuhellenden organischen Materialien können den verschiedenartigsten Verarbeitungszuständen (Rohstoffe, Halbfabrikate oder Fertigfabrikate) angehören. Sie können andererseits in Form der verschiedenartigsten geformten Gebilden vorliegen, d. h. beispielsweise als vorwiegend dreidimensional ausgedehnte Körper wie Platten, Profile, Spritzgußförmlinge, verschiedenartige Werkstücke, Schnitzel, Granulate oder Schaumstoffe, ferner als vorwiegend zweidimensional ausgebildete Körper wie Filme, Folien, Lacke, Überzüge, Imprägnierungen und Beschichtungen oder als vorwiegend eindimensional ausgebildete Körper wie Fäden, Fasern, Flocken, Drähte. Die besagten Materialien können andererseits auch in ungeformten Zuständen in den verschiedenartigsten homogenen oder inhomogenen Verteilungsformen, wie z. B. als Pulver, Lösungen, Emulsionen, Dispersionen, Latices, Pasten oder Wachse vorliegen.

Fasermaterialien können beispielsweise als endlose Fäden (verstreckt oder unverstreckt), Stapelfasern, Flocken, Strangware, textile Fäden, Garne, Zwirne, Faservliese, Filze, Watten, Beflockungs-Gebilde oder als textile Gewebe oder textile Verbundstoffe, Gewirke sowie als Papiere, Pappen oder Papiermassen vorliegen.

Den erfindungsgemäß anzuwendenden Verbindungen kommt u. a. Bedeutung für die Behandlung von textilen organischen Materialien, insbesondere textilen Geweben, wie z. B. als Pulver, Lösungen, Emulsionen, Dispersionen, Latices, Pasten oder Wachse vorliegen.

Fasermaterialien können beispielsweise als endlose Fäden (verstreckt oder unverstreckt), Stapelfasern, Flocken, Strangware, textile Fäden, Garne, Zwirne, Faservliese, Filze, Watten, Beflockungs-Gebilde oder als textile Gewebe oder textile Verbundstoffe, Gewirke sowie als Papiere, Pappen oder Papiermassen vorliegen.

Den erfindungsgemäß anzuwendenden Verbindungen kommt u. a. Bedeutung für die Behandlung von textilen organischen Materialien, insbesondere textilen Geweben, zu. Sofern Fasern, welche als Stapelfasern oder Endlosfäden, in Form von Strängen, Geweben, Gewirken, Vliesen, beflockten Substrate oder Verbindstoffen vorliegen können, erfindungsgemäß optisch aufzuhellen sind, geschieht dies mit Vorteil in wäßrigem Medium, worin die betreffenden Verbindungen in feinverteilter Form (Suspensionen), sogenannten Mikrodispersionen, gegebenenfalls Lösungen) vorliegen. Gegebenenfalls können bei der Behandlung Dispergier-, Stabilisier-, Netz- und weitere Hilfsmittel zugesetzt werden.

In Abhängigkeit vom verwendeten Aufheller-Verbindungstyp kann es sich als vorteilhaft erweisen, in neutralem oder alkalischem oder saurem Bade zu arbeiten. Die Behandlung wird üblicherweise bei Temperaturen von etwa 20 bis 140°C, beispielsweise bei Siedetemperatur des Bades oder in deren Nähe (etwa 90°C), durchgeführt. Für die erfindungsgemäße Veredelung textiler Substrate kommen auch Lösungen oder Emulsionen in organischen Lösungsmitteln in Betracht, wie dies in der Färbereipraxis in der sogenannten Lösungsmittelfärberei (Foulard-Thermofixierapplikation, Ausziehfärbeverfahren in Färbemaschinen) praktiziert wird.

Die neuen optischen Aufhellmittel gemäß vorliegender Erfindung können ferner den Materialien vor oder während deren Verformung zugesetzt bzw. einverleibt werden. So kann man sie beispielsweise bei der Herstellung von Filmen, Folien (z. B. Einwalzen in Polyvinylchlorid in der Hitze) oder Formkörpern der Preßmasse oder Spritzgußmasse beifügen.

Sofern die Formgebung voll- oder halbsynthetischer organischer Materialien durch Spinnverfahren bzw. über Spinnmassen erfolgt, können die optischen Aufheller nach folgenden Verfahren appliziert werden:

— Zugabe zu den Ausgangssubstanzen (z. B. Monomeren) oder Zwischenprodukten (z. B. Vorkondensaten, Präpolymeren), d. h. vor oder während der Polymerisation, Polykondensation oder Polyaddition,
— Aufpudern auf Polymerisatschnitzel oder Granulate für Spinnmassen,
— Badfärbung von Polymerisatschnitzel oder Granulaten für Spinnmassen,
— dosierte Zugabe zu Spinnschmelzen oder Spinnlösungen,
— Applikation auf Spinnkabel vor dem Verstrecken.

Die neuen optischen Aufhellmittel gemäß vorliegender Erfindung können beispielsweise auch in folgenden Anwendungsformen eingesetzt werden:

a) Mischungen mit Farbstoffen (Nuancierung) oder Pigmenten (Farb- oder insbesondere z. B.

Weißpigmenten) oder als Zusatz zu Färbebädern, Druck-, Ätz- oder Reservepasten. Ferner auch zur Nachbehandlung von Färbungen, Drucken oder Ätzdrucken,

b) in Mischungen mit sogenannten »Carriern«, Netzmitteln, Weichmachern, Quellmitteln, Antioxydantien, Lichtschutzmitteln, Hitzestabilisatoren, chemischen Bleichmitteln (Chlorid-Bleiche, Bleichbäder-Zusätze),

c) in Mischung mit Vernetzern, Appreturmitteln (z. B. Stärke oder synthetischen Appreturen) sowie in Kombination mit den verschiedensten Textilveredelungsverfahren, insbesondere Kunstharzausrüstungen (z. B. Knitterfest-Ausrüstungen wie »wash-and-wear«, »permanent-press«, »noniron«), ferner Flammfest-, Weichgriff-Schmutzablöse (»anti-soiling«)- oder Antistatisch-Ausrüstungen oder antimikrobiellen Ausrüstungen,

d) Einarbeiten der optischen Aufhellmittel in polymeren Trägermaterialien (Polymerisations-, Polykondensations- oder Polyadditionsprodukte) in gelöster oder dispergierter Form zur Anwendung z. B. in Beschichtungs-, Imprägnier- oder Bindemitteln (Lösungen, Dispersionen, Emulsionen) für Textilien, Vliese, Papier, Leder,

e) als Zusätze zu sogenannten »master batches«,

f) als Zusätze zu den verschiedensten industriellen Produkten, um dieselben marktfähiger zu machen (z. B. Aspektverbesserungen von Seifen, Waschmitteln, Pigmenten),

g) in Kombination mit anderen, optisch aufhellend wirkenden Substanzen,

h) in Spinnbadpräparationen, d. h. als Zusätze zu Spinnbädern, wie sie zur Gleitfähigkeitsverbesserung für die Weiterverarbeitung von Synthese-Fasern verwendet werden, oder aus einem speziellen Bad vor der Verstreckung der Faser,

i) als Scintillatoren, für verschiedene Zwecke photographischer Art, wie z. B. für elektrophotographische Reinproduktion oder Supersensibilisierung,

j) je nach Substitution als Laser-Farbstoffe.

Wird das Aufhellverfahren mit Textil-Behandlungs- oder Veredlungsmethoden kombiniert, so kann die kombinierte Behandlung in vielen Fällen vorteilhafterweise mit Hilfe entsprechender beständiger Präparate erfolgen, welche die optisch aufhellenden Verbindungen in solcher Konzentration enthalten, daß der gewünschte Aufhelleffekt erreicht wird.

In gewissen Fällen werden die Aufheller durch eine Nachbehandlung zur vollen Wirkung gebracht. Diese kann beispielsweise eine chemische (z. B. Säure-Behandlung), eine thermische (z. B. Hitze) oder eine kombinierte chemisch/thermische Behandlung darstellen. So verfährt man beispielsweise bei der optischen Aufhellung einer Reihe von Fasersubstraten, z. B. von Polyesterfasern, mit den erfindungsgemäßen Aufhellern zweckmäßig in der Weise, daß man diese Fasern mit den wäßrigen Dispersionen (gegebenenfalls auch Lösungen) der Aufhellmittel bei Temperaturen unter 75° C, z. B. bei Raumtemperatur, imprägniert und einer trockenen Wärmebehandlung bei Temperaturen über 100° C unterwirft, wobei es sich im allgemeinen empfiehlt, das Fasermaterial vorher noch bei mäßig erhöhter Temperatur, z. B. bei mindestens 60° C bis etwa 130° C zu trocknen. Die Wärmebehandlung in trockenem Zustande erfolgt dann vorteilhaft durch Erwärmen in einer Trockenkammer, durch Bügeln im angegebenen Temperaturintervall oder auch durch Behandeln mit trockenem, überhitztem Wasserdampf. Die Trocknung und trockene Wärmebehandlung können auch unmittelbar nacheinander ausgeführt oder in einem einzigen Arbeitsgang zusammengelegt werden.

Die Menge der erfindungsgemäß zu verwendenden neuen optischen Aufheller, bezogen auf das optisch aufzuhellende Material, kann in weiten Grenzen schwanken. Schon mit sehr geringen Mengen, in gewissen Fällen z. B. solchen von 0,001 Gewichtsprozent, kann ein deutlicher und haltbarer Effekt erzielt werden. Es können aber auch Mengen bis zu etwa 0,8 Gewichtsprozent und gegebenenfalls bis zu etwa 2 Gewichtsprozent zur Anwendung gelangen. Für die meisten praktischen Belange sind vorzugsweise Mengen zwischen 0,005 und 2, vorzugsweise 0,1 bis 0,5 Gewichtsprozent von Interesse.

Aus verschiedenen Gründen ist es oft zweckmäßig, die Aufheller nicht als solche, d. h. rein einzusetzen, sondern vermischt mit den verschiedensten Hilfs- und Coupiermitteln, wie z. B. wasserfreiem Natriumcarbonat, Natriumsulfat-decahydrat, Natriumchlorid, Natriumcarbonat, Alkalimetallphosphaten, wie Natrium- oder Kaliumorthophosphat, Natrium- oder Kaliumpyrophosphat und Natrium- oder Kaliumtripolyphosphate oder Alkalimetallsilikaten.

Die neuen optischen Aufhellmittel eignen sich auch besonders als Zusätze für Waschbäder oder zu Gewerbe- und Haushaltswaschmitteln, wobei sie in verschiedener Weise zugesetzt werden können. Zu Waschbädern werden sie zweckmäßig in Form ihrer Lösungen in Wasser oder organischen Lösungsmitteln oder auch in feiner Verteilung als wäßrige Dispersionen zugegeben. Zu Haushalt- oder gewerblichen Waschmitteln werden sie vorteilhaft in irgendeiner Phase des Herstellungsprozesses der Waschmittel, z. B. der sogenannten »Slurry« vor dem Zerstäuben dem Waschpulver oder bei der Vorbereitung flüssiger Waschmittelkombinationen zugesetzt. Die Zugabe kann sowohl in Form einer Lösung oder Dispersion in Wasser oder anderen Lösungsmitteln als auch ohne Hilfsmittel als trocknes Aufhellpulver erfolgen. Man kann die Aufhellmittel beispielsweise mit den waschaktiven Substanzen vermischen, verkneten oder vermahlen und so dem fertigen Waschpulver zumischen. Sie können jedoch auch gelöst oder vordispergiert auf das fertige Waschmittel aufgesprüht werden.

Als Waschmittel kommen die bekannten Mischungen von Waschaktivsubstanzen wie beispielsweise Seife in Form von Schnitzeln und Pulver, Synthetika, lösliche Salze von Sulfonsäurehalbestern höherer Fettalkohole, höher und/oder mehrfach alkylsubstituierter Arylsulfonsäuren, Sulfocarbonsäureester mittlerer bis höherer Alkohole, Fettsäureacylaminoalkyl-, oder -aminoacrylglycerinsulfonate, Phosphorsäureester von Fettalkoholen usw. in Frage. Als Aufbaustoffe, sogenannte »Builders«, kommen z. B. Alkalipoly- und -polymetaphosphate, Alkalipyrophosphate, Alkalisalze der Carboxymethylcellulose und andere »Soilredepositionsinhibitoren«, ferner Alkalisilikate, Alkalicarbonate, Alkaliborate, Alkaliperborate, Nitrilotriessigsäure, Äthylendiaminotetraessigsäure, Schaumstabilisatoren wie Alkanolamide höherer Fettsäuren, in Betracht. Ferner können in den Waschmitteln beispielsweise enthalten sein:

Antistatische Mittel, rückfettende Hautschutzmittel wie Lanolin, Enzyme, Antimikrobika, Parfüme und Farbstoffe.

Die neuen optischen Aufheller haben den besonderen Vorteil, daß sie auch bei Gegenwart von Aktivchlorspendern, wie z. B. Hypochlorid, wirksam sind und ohne wesentliche Einbuße der Effekte in Waschbädern mit nichtionogenen Waschmittel, wie z. B. Alkylphenolpolyglykoläthern, verwendet werden können.

Die erfindungsgemäßen Verbindungen werden in Mengen von 0,005—1% oder mehr, bezogen auf das Gewicht des flüssigen oder pulverförmigen, fertigen Waschmittels, zugesetzt. Waschflotten, die die angegebenen Mengen der beanspruchten optischen Aufheller enthalten, verleihen beim Waschen von Textilien aus Cellulosefasern, Polyesterfasern, Wolle etc. einen brillanten Aspekt am Tageslicht.

Die Waschbehandlung wird beispielsweise wie folgt durchgeführt:

Die angegebenen Textilien werden während 1 bis 30 Minuten bei 20 bis 100°C in einem Waschbad behandelt, das 1 bis 10 g/kg eines aufgebauten, zusammengesetzten Waschmittels und 0,05 bis 1%, bezogen auf das Waschmittelgewicht, der beanspruchten Aufhellmittel enthält. Das Flottenverhältnis kann 1 : 3 bis 1 : 50 betragen. Nach dem Waschen wird wie üblich gespült und getrocknet. Das Waschbad kann als Bleichzusatz 0,2 g/l Aktivchlor (z. B. als Hypochlorid) oder 0,1 bis 2 g/l Natriumperborat enthalten.

In den folgenden Beispielen sind Prozente immer Gewichtsprozente; Schmelz- und Siedepunkte sind, sofern nicht anders vermerkt, unkorrigiert.

## Beispiel 1

3,6 g Carbonsäurechlorid der Formel

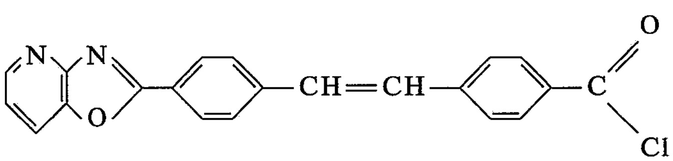

(15)

werden unter gutem Rühren bei Raumtemperatur in eine Lösung von 1,0 g Acetamidoxim und 1 ml Pyridin in 50 ml Toluol eingetragen und noch 1 Stunde bei Raumtemperatur gerührt. Anschließend wird 2 Stunden auf 80°C erhitzt, abgekühlt und abgesaugt, halogenfrei gewaschen und getrocknet. Man erhält 3,4 g Acylaminoverbindung, welche zum Ringschluß in 40 ml Dimethylformamid 4 Stunden am Rückfluß gekocht werden. Beim Abkühlen kristallisiert das 1.2.4-Oxadiazol aus. Nach Absaugen, Waschen mit Methanol und Wasser und Trocknen erhält man 3,2 g der Verbindung der Formel

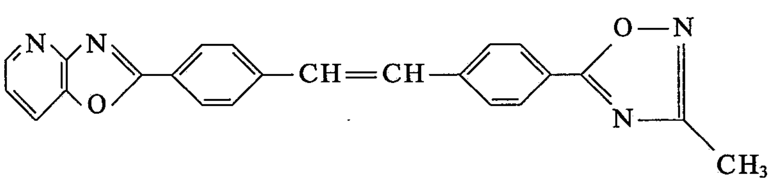

(16)

Aus o-Dichlorbenzol schwach gelbliche Kristalle vom Schmelzpunkt 300°C Ultraviolett-Absorptionsmaximum bei $\lambda$ = 367 mm (gemessen in DMF).

Das als Ausgangsmaterial verwandte Säurechlorid (15) kann nach folgenden Vorschriften hergestellt werden:

15,7 g der Verbindung der Formel, dargestellt z. B. nach Vorschriften der DE-OS 1 294 917

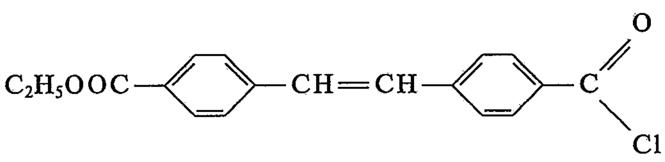

(17)

werden im schwachen Stickstoffstrom mit 250 ml trockenem Xylol und 5,5 g 2-Amino-3-hydroxypyridin bei 60° C verrührt. In dieser Mischung tropft man langsam 7 ml Triäthylamin und erwärmt anschließend 1 Stunde auf Rückflußtemperatur. Nach Abkühlen der Reaktionsmischung wird das Produkt abgesaugt und mit Methanol und Wasser gewaschen. Nach dem Trocknen erhält man 15,9 g Acylaminoverbindung der Formel

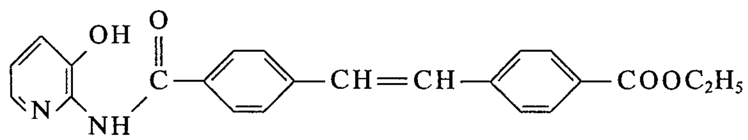

(18)

Diese werden in 150 ml Tetralin suspendiert und unter Zugabe von 0,5 g p-Toluolsulfonsäure in einem schwachen Stickstoffstrom an einem Wasserabscheider zur Rückflußtemperatur erhitzt. Nach 8 Stunden ist die Acylaminoverbindung in einer Probe dünnschichtchromatographisch nicht mehr nachweisbar. Es wird 1 g Aktivkohle in die Reaktionsmischung gegeben und nach 15 Minuten heiß filtriert. Nach Absaugen und Waschen des Produkts mit Tetralin und Methanol erhält man 12,5 g der Verbindung der Formel

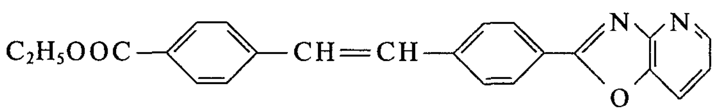

(19)

Zweimaliges Umkristallisieren aus Xylol unter Zuhilfenahme von Aktivkohle ergibt schwach gelbliche Kristalle vom Schmelzpunkt 267° C Ultraviolett-Absorptionsmaximum bei $\lambda$ = 359 mm (gemessen in DMF).

4,0 g der Verbindung der Formel

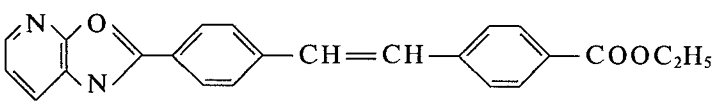

werden in 30 ml Dioxan mit 2 ml 10 N NaOH 5 Stunden bei 85—90° C gerührt, anschließend wird mit 10 ml Methanol verdünnt; nach Abkühlen auf Raumtemperatur wird abgesaugt und mit Methanol gewaschen. Das Nutschgut wird feucht in 100 ml Dimethylformamid aufgenommen und bei 100° C mit ca. 5 ml konzentrierter Salzsäure versetzt, nach Abkühlen wird abgesaugt, neutral gewaschen und getrocknet. Man erhält 3,1 g der Verbindung der Formel

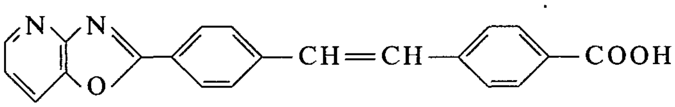

Aus dieser Carbonsäure erhält man durch 3stündiges Kochen in überschüssigem Thionylchlorid das Säurechlorid der Formel

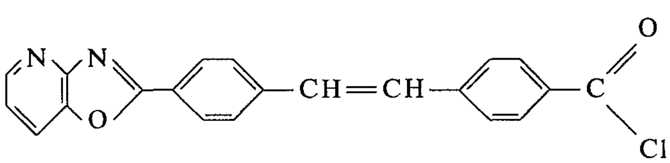

(15)

## Beispiel 2

3,6 g Carbonsäurechlorid der Formel (15) werden unter gutem Rühren bei Raumtemperatur in ein Gemisch aus 1,4 g Benzoesäurehydrazid, 1,0 ml Pyridin und 50 ml Toluol eingetragen. Nach einstündigem Rühren bei Raumtemperatur wird der Ansatz noch 2 Stunden auf 85—90° C erwärmt, danach wird abgekühlt, abgesaugt und zunächst mit Methanol, dann mit Wasser halogenfrei gewaschen und getrocknet. Der Rückstand wird in 30 ml Thionylchlorid eingetragen und 20 Stunden auf Rückflußtemperatur gehalten. Der Thionylchloridüberschuß wird abdestilliert und zuletzt der verbleibende Rückstand mit Eiswasser ausgerührt, abgesaugt und neutral gewaschen. Nach dem Trocknen verbleiben 2,7 g der Verbindung der Formel

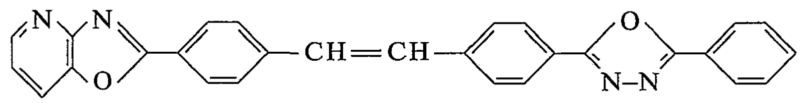 (20)

die nach dem Umkristallisieren aus Dimethylformamid unter Zuhilfenahme von Bleicherde schwach gelbliche Kristalle von Tp. 287—288°C ergeben. Ultraviolett-Absorptionsmaximum bei $\lambda$ = 372 mm (gemessen in DMF).

## Beispiel 3

3,6 g Carbonsäurechlorid der Formel (15) werden unter gutem Rühren bei Raumtemperatur in eine Mischung und 50 ml Methanol und 1,0 ml Pyridin eingetragen. Nach einstündigem Rühren bei Raumtemperatur wird noch 1 Stunde zum Sieden erhitzt, danach wird abgekühlt, abgesaugt und mit Methanol, dann mit Wasser halogenfrei gewaschen. Nach dem Trocknen erhält man 3,0 g der Verbindung der Formel

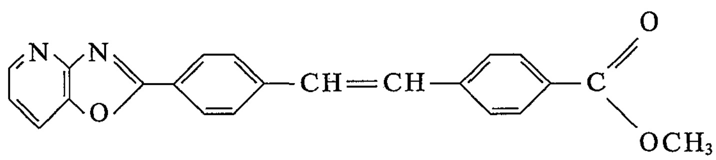 (21)

Aus o-Dichlorbenzol fast farblose Kristalle vom Schmelzpunkt 262—266° C. Ultraviolett-Absorptionsmaximum bei $\lambda$ = 358 mm (gemessen in DMF).

## Beispiel 4

3,6 g Carbonsäurechlorid der Formel (15) werden bei guter Rührung und Eiskühlung in eine Mischung aus 20 ml Dimethylformamid und 20 ml konzentriertem Ammoniak eingetragen, nach Abklingen der exothermen Reaktion nach 1 Stunde bei Raumtemperatur, danach noch 1 Stunde bei 95—100° C gerührt. Das Produkt wird kalt abgesaugt und neutral gewaschen. Nach dem Trocknen erhält man 2,9 g Carbonsäureamid der Formel

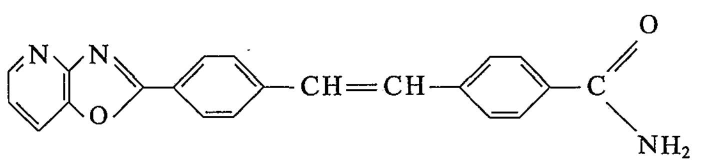 (22)

Diese werden in 25 ml Thionylchlorid 20 Stunden am Rückfluß gekocht. Danach zeigt eine der Reaktionsmischung entnommene Probe im IR-Spektrum keine Carbonyl-Bande mehr. Der Thionylchlorid-Überschuß wird im Vakuum abdestilliert, der Rückstand wird in Eiswasser ausgerührt, abgesaugt und neutral gewaschen. Nach Trocknen erhält man 1,6 g Nitril der Formel

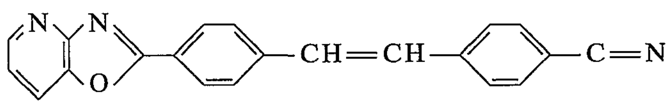 (23)

Umkristallisieren aus o-Dichlorbenzol unter Zuhilfenahme von Bleicherde liefert schwach gelbliche Kristalle vom Schmelzpunkt 297—298° C. Ultraviolett-Absorptionsmaximum bei $\lambda$ = 342 mm (gemessen in DMF).

## Beispiel 5

4,0 g der Verbindung der Formel

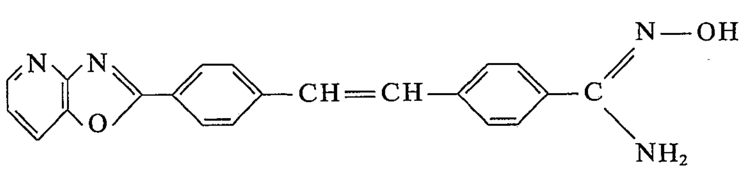 (24)

werden bei Raumtemperatur mit 1,0 ml Pyridin und 50 ml Toluol verrührt. In diese Mischung wird 0,75 ml Acetylchlorid, verdünnt mit 5 ml Toluol, eingetropft und noch 1 Stunde bei Raumtemperatur, eine weitere Stunde bei 75°C gerührt. Das entstandene Produkt wird kalt abgesaugt, mit Methanol gewaschen und bei Raumtemperatur getrocknet. Es wird in 25 ml Dimethylformamid 4 Stunden am Rückfluß gekocht, kalt abgesaugt und mit Methanol gewaschen. Es verbleiben 2,3 g der Verbindung der Formel

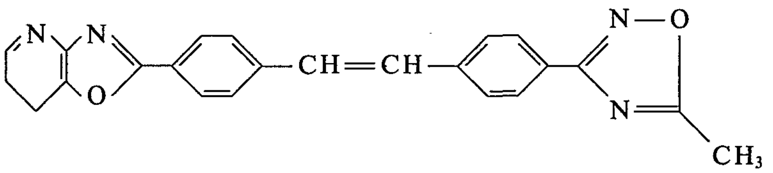

(25)

Durch Umkristallisieren aus Dimethylformamid unter Zuhilfenahme von Aktivkohle erhält man schwach gelbliche Kristalle. Die Verbindung besitzt im Ultraviolett-Spektrum ein Absorptionsmaximum bei 364 mm (gemessen in DMF).

Die Verbindung der Formel (24) erhält man wie folgt:

6,4 g des Nitrils der Formel (23) werden in 250 ml i-Propanol mit 2,8 g Hydroxylammoniumhydrochlorid und 3,3 g wasserfreiem Natriumacetat 10 Stunden am Rückfluß erhitzt. Nach dem Erkalten wird auf 1500 ml Eiswasser gegeben, abgesaugt und mit Wasser gewaschen. Man erhält nach Trocknen bei Raumtemperatur 4,8 g der Verbindung (24).

## Beispiel 6

15,7 g der Verbindung der Formel

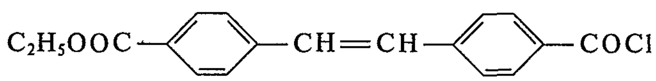

werden im schwachen Stickstoffstrom mit 250 ml Xylol und 6,4 g 3-Amino-2-chlorpyridin bei Rückflußtemperatur verrührt, bis die Salzsäureentwicklung beendet ist. Es wird kalt abgesaugt und mit Wasser chloridionenfrei gewaschen. Nach dem Trocknen erhält man 14,6 g der Acylaminoverbindung der Formel

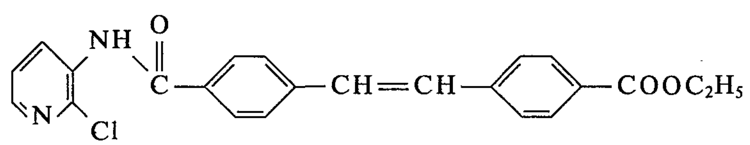

vom Schmelzpunkt 212° C.

Diese werden in einer Mischung aus 300 ml Dimethylformamid, 100 ml Pyridin, 20 g wasserfreiem Kupfer-II-acetat und 3,8 g Zinkpulver 6 Stunden bei Rückflußtemperatur gerührt, anschließend wird heiß filtriert. Aus der Lösung kristallisieren über Nacht 5,5 g der Verbindung der Formel

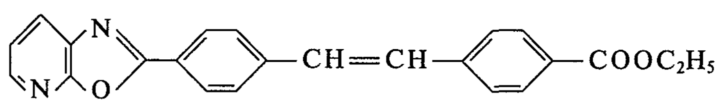

aus. Durch Umkristallisieren aus Pyridin unter Zuhilfenahme von Aktivkohle erhält man schwach gelbliche Kristalle vom Schmelzpunkt 260° C. Ultraviolett-Absorptionsmaximum (gemessen in DMF) bei $\lambda = 334$ mm.

4,0 g der Verbindung der Formel

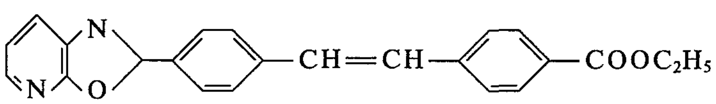

werden in 30 ml Dioxan mit 2 ml 10 N NaOH 5 Stunden bei 85–90° C gerührt, anschließend wird mit 10 ml Methanol verdünnt; nach Abkühlen auf Raumtemperatur wird abgesaugt und mit Methanol gewaschen. Das Nutschgut wird feucht in 100 ml Dimethylformamid aufgenommen und bei 100° C mit ca. 5 ml konzentrierter Salzsäure versetzt, nach Abkühlen wird abgesaugt, neutral gewaschen und getrocknet. Man erhält 3,0 g der Verbindung der Formel

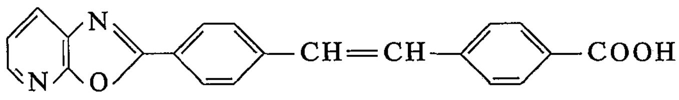

Aus dieser Carbonsäure erhält man durch 3stündiges Kochen in überschüssigem Thionylchlorid das Säurechlorid der Formel

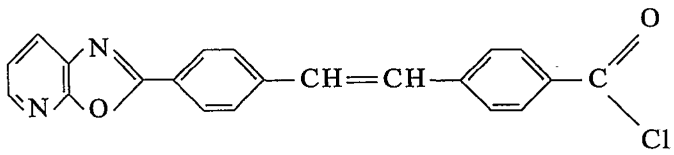

Aus diesem Säurechlorid erhält man analog zu den oben beschriebenen Verfahren die folgenden Verbindungen:

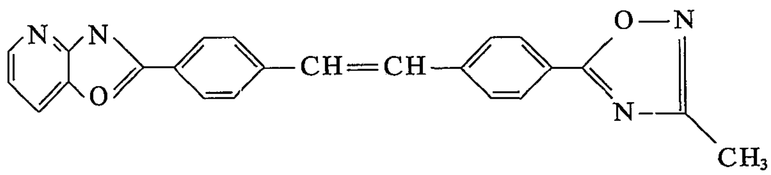

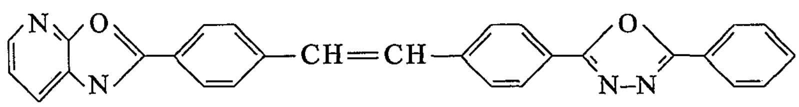

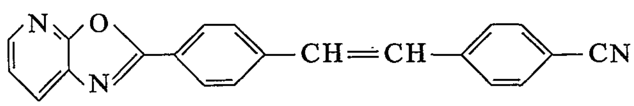

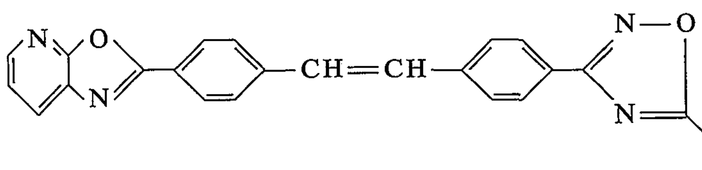

Beispiel 7

2,75 g des Phosphoniumbromids der Formel

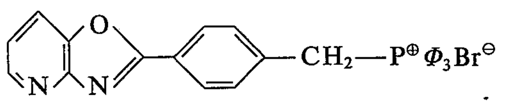

(26)

**werden unter Stickstoffatmosphäre in 50 ml Dimethylformamid unter leichtem Erwärmen gelöst. Bei 30–35° C werden 0,56 g Kalium-tert.-butylat eingetragen und 5 Minuten durchgerührt. In die violette Lösung wird innerhalb von 10 Minuten eine Lösung von 0,82 g p-Carbmethoxybenzaldehyd in 10 ml Dimethylformamid eingetropft und anschließend noch 4 Stunden bei 100° C gehalten. Beim Abkühlen kristallisieren 1,2 g Verbindung der Formel**

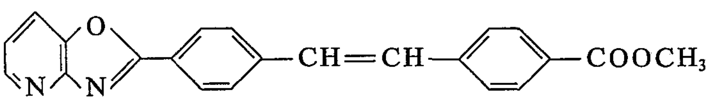

**Das als Ausgangsmaterial verwendete Phosphoniumbromid (26) kann wie folgt hergestellt werden:**

**110 g 2-Amino-3-hydroxypyridin, 136 g 4-Methylbenzoesäure und 3 g Borsäure werden in 1000 ml Tetralin in eine mit einem Wasserabscheider ausgerüstete Apparatur unter Stickstoffatmosphäre zunächst 1 Stunde auf 150°, dann 2 Stunden auf 170° und schließlich 12 Stunden bei 200–210° C verrührt. In der Reaktionszeit scheiden sich insgesamt ca. 28–30 ml Wasser ab. Beim Abkühlen kristallisiert das Produkt in Form kräftiger Nadeln aus. Durch Absaugen und Waschen mit Methanol und anschließend mit Wasser erhält man 141 g 2-(4-Methylphenyl)-pyridin-(b-4.5)oxazol vom**

Schmelzpunkt 141° C.

30 g 2-(4-Methylphenyl)-pyridin-(b-4.5)-oxazol werden unter leichtem Erwärmen in 750 ml absolutem Tetrachlorkohlenstoff gelöst. Nach Zugabe von 26 g N-Bromsuccinimid und 1 g Dibenzoylperoxid wird 5 Stunden am Rückfluß gekocht, anschließend heiß vom ausgefallenen Succinimid abfiltriert. Beim Abkühlen erhält man aus der Mutterlauge 21,0 g (48%) 2-(4-Brommethylphenyl)-pyridin-(b-4.5)oxazol. Aus Äthylacetat farblose Kristalle vom Zersetzungspunkt 240 – 243° C.

13,1 g Triphenylphosphin werden in 30 ml Benzol unter Erwärmen gelöst und anschließend zu einer Lösung von 14,4 g 2-(4-Brommethylphenyl)-pyridin-oxazol in 150 ml Benzol bei 50° gegeben. Während des 12stündigen Rührens bei Raumtemperatur und 8stündigen Rührens bei 50° fällt das Reaktionsprodukt aus. Es wird kalt abgesaugt und mit Benzol gewaschen. Man erhält 22,3 g (81%) des Phosphoniumbromids (26) vom Schmelzpunkt 298 – 299° C.

Durch Umsetzen des Phosphoniumbromids (26) mit 4-Cyanbenzaldehyd erhält man in analoger Weise die Verbindung der Formel

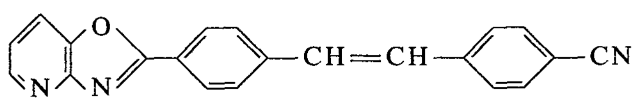

## Beispiel 8

Unter einer Stickstoffatmosphäre werden 2,8 g fein gepulvertes Kaliumhydroxid in 40 ml Dimethylformamid suspendiert. Unter leichter Kühlung wird eine Lösung von 2,6 g der Verbindung der Formel

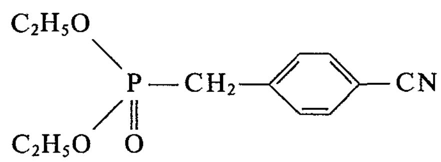

(27)

und 2,5 g der Verbindung der Formel

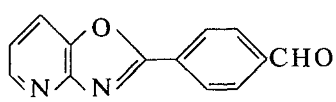

(28)

in 40 ml Dimethylformamid eingetropft. Die Temperatur des Reaktionsgemisches soll dabei 25° C nicht übersteigen. Nach 20stündigem Rühren bei Raumtemperatur wird auf 100 ml Eiswasser, welches 4 ml konzentrierte Salzsäure enthält, gegossen, kurz auf 90° C erwärmt und nach dem Erkalten abgesaugt. Nach Neutralwaschen und Trocknen erhält man 1,8 g der Verbindung der Formel

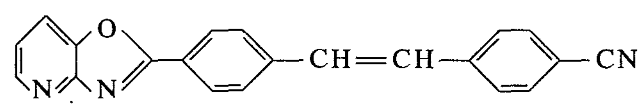

Den Aldehyd der Formel (28) erhält man nach folgender Vorschrift:

Eine Lösung von 14,5 g (50 mMol) 2-[4-Brommethylphenyl]-pyridin-[b-4.5]-oxazol in 250 ml Chloroform wird mit einer Lösung von 21,0 g (150 mMol) Urotropin in 250 ml Chloroform vereinigt und 24 Stunden am Rückfluß gekocht. Der entstandene Niederschlag wird kalt abgesaugt und mit Chloroform gewaschen. Nach dem Trocknen wird der Feststoff pulverisiert und in eine Mischung aus 135 ml Eisessig und 135 ml Wasser eingetragen und 2 Stunden auf Rückflußtemperatur erhitzt. Nach dem Abkühlen wird der auskristallisierte Aldehyd abgesaugt und neutral gewaschen.

Man erhält 3,8 g (35%) Pyridin-[b-4.5]-oxazolyl-2-benzaldehyd (28) vom Fp. 206° C.

## Beispiel 9

2,75 g des Phosphoniumbromids der Formel

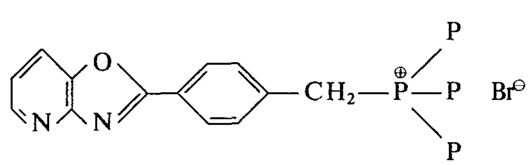

(26)

werden unter Stickstoffatmosphäre in 50 ml Dimethylformamid unter leichtem Erwärmen gelöst. Bei 30 – 35° C werden 0,56 g Kalium-tert.-butylat eingetragen und 5 Minuten nachgerührt. In die violette Lösung wird innerhalb von 10 Minuten eine Lösung von 1,25 g des Aldehyds der Formel

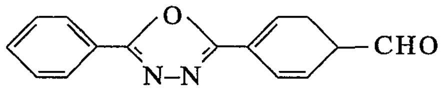 (29)

eingetropft und anschließend noch 4 Stunden bei 100°C gehalten. Beim Abkühlen kristallisieren 1,05 g der Verbindung der Formel

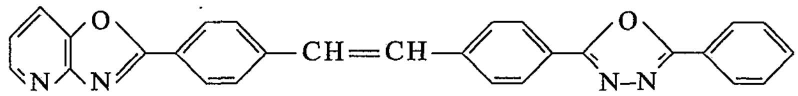

Die Verbindung (29) erhält man wie folgt:

23,6 g 2-Phenyl-5-(4-methylphenyl)-1.3.4-oxadiazol werden in 200 ml absolutem Tetrachlorkohlenstoff unter leichtem Erwärmen gelöst. Es werden 17,8 g N-Bromsuccinimid und 0,2 g Azoisobuttersäure zugegeben und 4 Stunden am Rückfluß gekocht. Es wird heiß abfiltriert, aus dem Filtrat kristallisieren beim Abkühlen 20,2 g der Verbindung der Formel

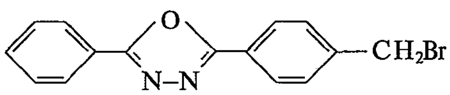

Aus Essigsäureäthylester erhält man farblose Kristalle vom Schmelzpunkt 170 – 172° C.

15,7 g dieser Brommethylverbindung werden in 250 ml Chloroform gelöst und mit einer Lösung von 21 g Urotropin in 250 ml Chloroform vereinigt. Die Mischung wird 24 Stunden bei Rückflußtemperatur gehalten, der ausgefallene Niederschlag wird kalt abgesaugt, mit Chloroform gewaschen und getrocknet. Der Rückstand wird in fein pulverisierter Form in einem Gemisch aus 135 ml Eisessig und 135 ml Wasser 2 Stunden bei Rückflußtemperatur gehalten. Beim Abkühlen kristallisieren 7,2 g der Verbindung der Formel

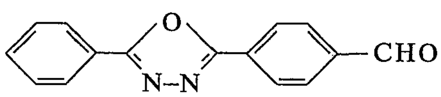

aus. Aus Äthanol erhält man farblose Kristalle vom Schmelzpunkt 275 – 280° C.

**Patentansprüche**

1. Verbindungen der allgemeinen Formel

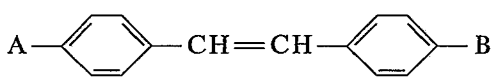

wobei A eine Gruppe der Formeln

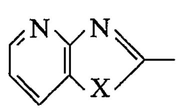 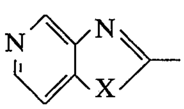 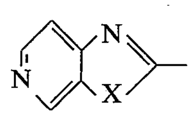

oder

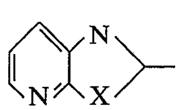

bedeutet, deren Pyridinring durch Fluor- oder Chloratome oder $C_1 - C_4$-Alkylgruppen substituiert sein kann und B eine Gruppe der Formeln

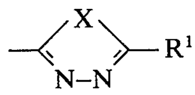 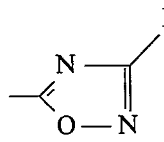 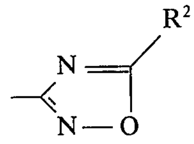

CN, $COOR^3$ oder 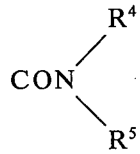

ist, wobei $R^1$ einen Phenylring, der durch 1 oder 2 Chloratome, ein oder zwei $C_1-C_4$-Alkyl- oder $C_1-C_4$-Alkoxy-$C_1-C_4$-alkylgruppen substituiert sein kann, $R^2$, $R^3$, $R^4$ und $R^5$ die gleiche Bedeutung wie $R^1$ haben und zusätzlich jeweils eine $C_1-C_{18}$-Alkylgruppe, eine Benzyl-, $C_1-C_6$-Chloralkyl-, $C_1-C_4$-Alkoxy-$C_1-C_4$-alkyl- oder $C_1-C_4$-Hydroxyalkylgruppe oder eine Gruppe der Formel $-(CH_2CH_2O)_n-R'$ mit $R'$=Wasserstoff oder $C_1-C_4$-Alkyl und $n=1$ oder 2 bedeuten, $R^3$, $R^4$ und $R^5$ zusätzlich auch ein Wasserstoffatom bedeuten und $R^4$ und $R^5$ zusammen mit dem Stickstoffatom auch einen Morpholin-, Piperidin- oder Piperazinring bedeuten und X ein Sauerstoff- oder Schwefelatom bedeutet.

2. Verbindungen nach Anspruch 1, dadurch gekennzeichnet, daß X ein Sauerstoffatom bedeutet.

3. Verbindungen nach Anspruch 1 der allgemeinen Formeln

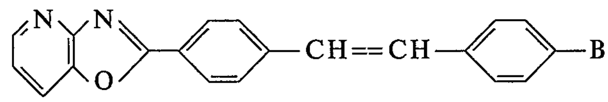

oder

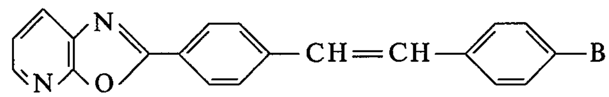

mit der in Anspruch 1 angegebenen Bedeutung für B, wobei $R^1$ einen Phenylring, der durch ein oder zwei Chloratome, eine oder zwei $C_1-C_4$-Alkyl- oder $C_1-C_4$-Alkoxy-$C_1-C_4$-alkylgruppen substituiert sein kann, $R^2$, $R^3$, $R^4$ und $R^5$ die gleiche Bedeutung wie $R^1$ haben und zusätzlich jeweils eine $C_1-C_6$-Alkyl-, $C_1-C_6$-Chloralkyl-, $C_1-C_4$-Alkoxy-$C_1-C_4$-alkyl-, $C_1-C_4$-Hydroxyalkyl-, Benzylgruppe oder eine Gruppe der Formel $-(CH_2CH_2O)_n-R'$ mit $n=1$ oder 2 und $R'$=Wasserstoff oder $C_1-C_4$-Alkyl bedeuten und $R^3$, $R^4$ und $R^5$ zusätzlich auch ein Wasserstoffatom bedeuten können.

4. Verbindungen nach Anspruch 1 der allgemeinen Formeln

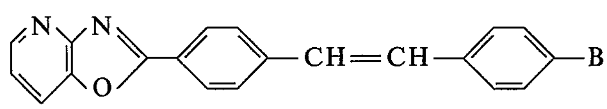

oder

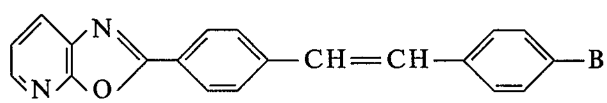

mit der in Anspruch 1 angegebenen Bedeutung für B, wobei $R^1$ Phenyl, mono- oder di-$C_1-C_2$-Alkylphenyl, $C_1-C_2$-Alkoxyphenyl, Chlorphenyl oder Dichlorphenyl, $R^2$, $R^3$, $R^4$ und $R^5$ die gleiche Bedeutung wie $R^1$ haben und zusätzlich jeweils $C_1-C_5$-Alkyl, $C_1-C_2$-Chloralkyl, $C_1-C_2$-Hydroxyalkyl, $C_1-C_2$-Alkoxy-$C_1-C_2$-alkyl oder Benzyl und $R^3$, $R^4$ und $R^5$ auch ein Wasserstoffatom bedeuten können.

5. Verfahren zur Herstellung der Verbindung nach Anspruch 1 bis 4, dadurch gekennzeichnet, daß man

a) wenn B eine Gruppe der Formel $-COOR^3$ oder

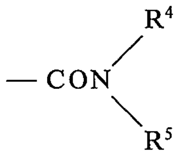

bedeutet, eine Carbonsäure der allgemeinen Formel

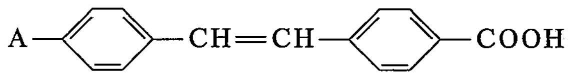

oder ein funktionelles Derivat dieser Säure mit einer Verbindung der Formel

$HOR^3$ oder 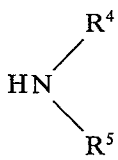

umsetzt, oder

b) wenn B eine Cyanogruppe bedeutet, aus einer Verbindung der allgemeinen Formel

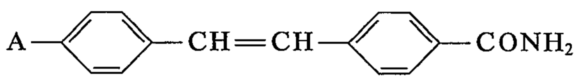

Wasser abspaltet, oder

c) wenn B eine Gruppe der Formel

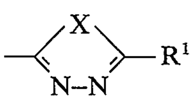

bedeutet, entweder eine Säure der Formel

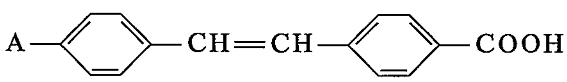

oder ein funktionelles Derivat dieser Säure mit einem Carbonsäurehydrazid der Formel

$H_2NNHCOR^1$

oder eine Säure der Formel

$R^1—COOH$

oder ein funktionelles Derivat dieser Säure mit einer Verbindung der Formel

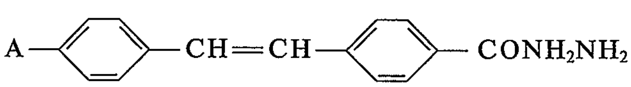

umsetzt und anschließend mit einem wasserabspaltenden Mittel cyclisiert, wobei, wenn X ein Schwefelatom bedeutet, die Cyclisierung mit $P_2S_5$ erfolgt, oder

d) wenn B eine Gruppe der Formel

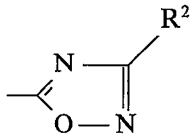

bedeutet, eine Säure der Formel

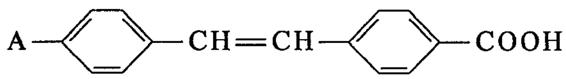

oder ein funktionelles Derivat dieser Säure mit einem Amidoxim der Formel

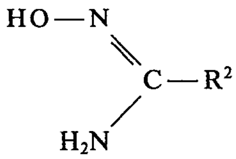

umsetzt und mit einem wasserabspaltenden Mittel cyclisiert oder

e) wenn B eine Gruppe der Formel

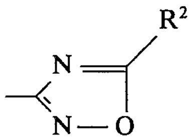

bedeutet, eine Verbindung der Formel

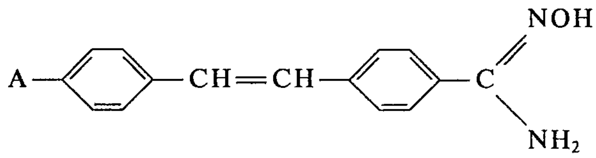

mit einem Carbonsäurechlorid der Formel

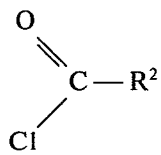

umsetzt und mit einem wasserabspaltenden Mittel cyclisiert, oder

f) eine Verbindung der Formel

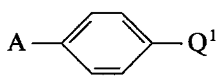

mit einer Verbindung der Formel

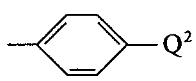

umsetzt, wobei eines der Symbole $Q^1$ oder $Q^2$ eine Aldehydgruppe und das andere Symbol eine Gruppe der Formeln

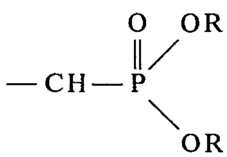

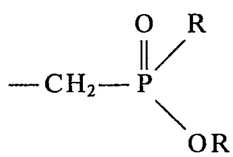

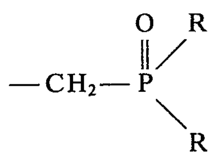

oder

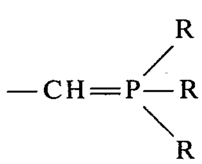

bedeutet, wobei R einen gegebenenfalls substituierten Alkylrest mit vorzugsweise 1–6 C-Atomen, einen Arylrest, vorzugsweise Phenyl, einen Cycloalkylrest oder einen Aralkylrest, vorzugsweise Benzyl, darstellt.

6. Verwendung der Verbindungen gemäß Anspruch 1 bis 4 als optische Aufheller.

## Claims

1. Compounds of the general formula

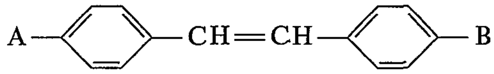

in which A represents a group of the formulae

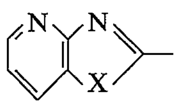
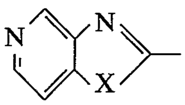
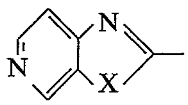

or

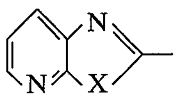

the pyridine ring of which may be substituted by fluorine or chlorine atoms or $C_1-C_4$-alkyl groups and B is a group of the formulae

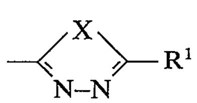
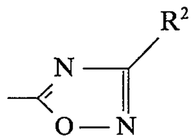
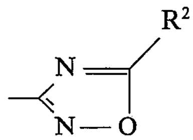

CN, $COOR^3$ or 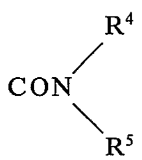

in which $R^1$ is a phenyl ring which may be substituted by 1 or 2 chlorine atoms, one or two $C_1-C_4$-alkyl or $C_1-C_4$-alkoxy-$C_1-C_4$-alkyl groups, $R^2$, $R^3$, $R^4$ and $R^5$ are defined as $R^1$ and additionally each represent a $C_1-C_{18}$-alkyl, benzyl, $C_1-C_6$-chloralkyl, $C_1-C_4$-alkoxy-$C_1-C_4$-alkyl or a $C_1-C_4$-hydroxyalkyl group or a group of the formula $-(CH_2CH_2O)_n-R'$ with R′ being hydrogen or $C_1$ to $C_4$-alkyl, and n being 1 or 2, $R^3$, $R^4$ and $R^5$ additionally also stand for hydrogen, and $R^4$ and $R^5$ together with the nitrogen atom also represent a morpholine, piperidine or piperazine ring, and X is an oxygen or sulfur atom.

2. Compounds as claimed in claim 1, wherein X is oxygen.

3. Compounds as claimed in claim 1 of the general formulae

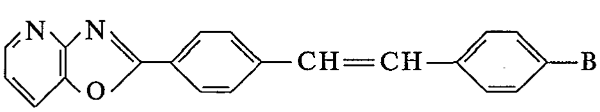

or

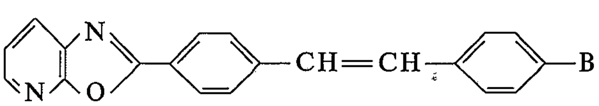

in which B is defined as in claim 1, wherein $R^1$ represents a phenyl ring optionally substituted by 1 or 2 chlorine atoms, 1 or 2 $C_1$ to $C_4$-alkyl or $C_1$ to $C_4$-alkoxy-$C_{1-4}$-alkyl groups, $R^2$, $R^3$, $R^4$ und $R^5$ are defined as $R^1$ and additionally each represent $C_{1-6}$-alkyl, $C_{1-6}$-chloroalkyl, $C_{1-4}$-alkoxy-$C_{1-4}$-alkyl, $C_{1-4}$-hydroxyalkyl, benzyl or a group of the formula $-(CH_2CH_2O)_n-R'$ with n being 1 or 2 and R′ being hydrogen or $C_{1-4}$-alkyl, and $R^3$, $R^4$ and $R^5$ in addition may stand for hydrogen.

4. Compounds as claimed in claim 1 of the general formulae

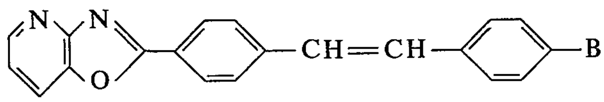

or

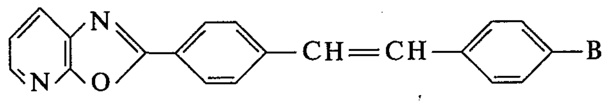

in which B is defined as in claim 1, in which $R^1$ is phenyl, mono- or di-$C_{1-2}$-alkylphenyl, $C_{1-2}$-alkoxyphenyl, chlorophenyl or dichlorophenyl, $R^2$, $R^3$, $R^4$ and $R^5$ are defined as $R^1$ and additionally each represent $C_{1-5}$-alkyl, $C_{1-2}$-chloroalkyl, $C_{1-2}$-hydroxyalkyl, $C_{1-2}$-alkoxy-$C_{1-2}$-alkyl or benzyl, and $R^3$, $R^4$ and $R^5$ may besides represent hydrogen.

5. Process for preparing the compound as claimed in claims 1 through 4, which comprises

a) – if B represents a group of the formula $-COOR^3$ or

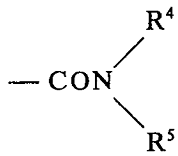

– reacting a carboxylic acid of the general formula

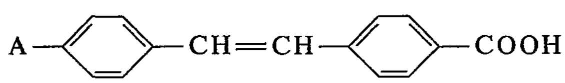

or a functional derivative of this acid with a compound of the general formula

$HOR^3$ or 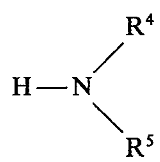

or

b) – if B represents a cyano group – splitting off water from a compound of the general formula

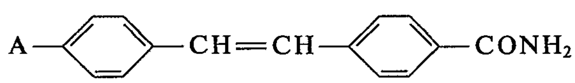

or

c) – if B represents a group of the formula

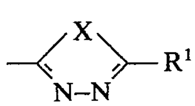

reacting either an acid of the formula

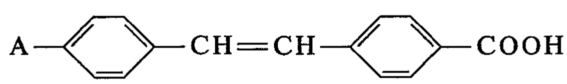

or a functional derivative of this acid with a carboxylic acid hydrazide of the formula

$H_2NNHCOR^1$

or an acid of the formula

$R^1—COOH$

or a functional derivative of this acid with a compound of the formula

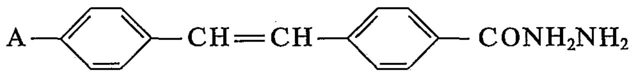

and subsequently cyclisizing the product obtained with an agent splitting off water, in which process — if X is sulfur — the cyclization is effected with $P_2S_5$, or

d) — if B represents a group of the formula

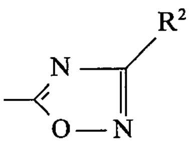

reacting an acid of the foumula

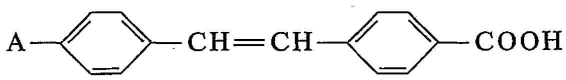

or a functional derivative of this acid with an amidoxime of the formula

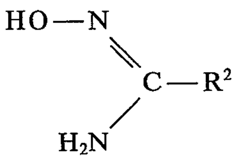

and cyclisizing the product obtained with an agent splitting off water, or

e) — if B represents a group of formula

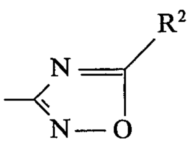

reacting a compound of the formula

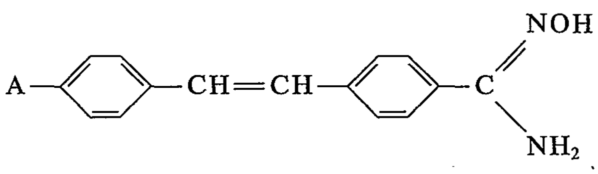

with a carboxylic acid chloride of the formula

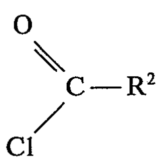

and cyclisizing the product obtained with an agent splitting off water, or

f) reacting a compound of the formula

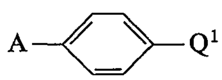

with a compound of the formula

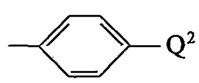

one of the symbols $Q^1$ and $Q^2$ representing an aldehyde group and the other symbol standing for a group of the formulae

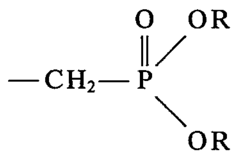 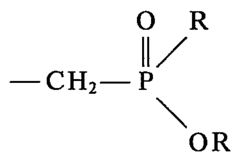 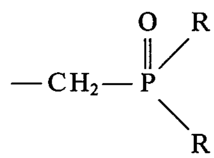

or

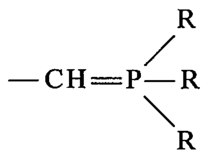

in which R is an optionally substituted alkyl with preferably 1 to 6 carbon atoms, an aryl radical, preferably phenyl, a cycloalkyl radical or an aralkyl radical, preferably benzyl.

**6. Use of the compounds according to claims 1 to 4 as optical brighteners.**

## Revendications

**1. Composés répondant à la formule générale:**

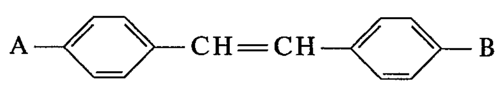

dans laquelle A représente un radical répondant à l'une des formules suivantes:

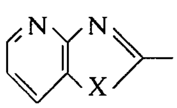 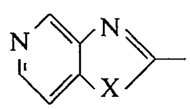 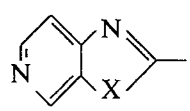

et

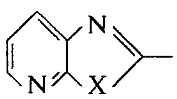

dont le noyau pyridinique peut porter comme substituants des atomes de fluor ou de chlore ou des radicaux alkyles en $C_1-C_4$, et B représente un radical répondant à l'une des formules suivantes:

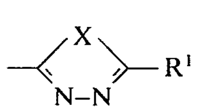 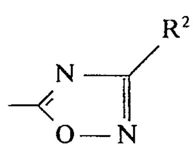 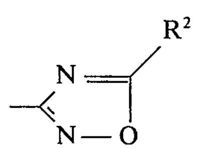

CN, $COOR^3$ et 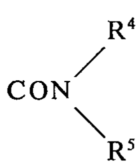

dans lesquelles $R^1$ représente un noyau phényle éventuellement porteur d'un ou deux atomes de chlore ou d'un ou deux radicaux alkyles en $C_1-C_4$ ou $C_1-C_4$-alcoxy-$C_1-C_4$-alkyles, $R^2$, $R^3$, $R^4$ et $R^5$ ont la même signification que $R^1$ et peuvent en outre représenter chacun un radical alkyle en $C_1-C_{18}$, benzyle, chloralkyle en $C_1-C_6$, $C_1-C_4$-alcoxy-$C_1-C_4$-alkyle ou hydroxyalkyle en $C_1-C_4$ ou un radical $-(CH_2CH_2O)_n-R'$ dans lequel R' désigne l'hydrogène ou un alkyle en $C_1-C_4$ et n est égal à 1 ou à 2, $R^3$, $R^4$ et $R^5$ peuvent en outre représenter chacun un atome d'hydrogène, $R^4$ et $R^5$ peuvent aussi former ensemble, et avec l'atome d'azote, un noyau de morpholine, de pipéridine ou de pipérazine, et X

représente un atome d'oxygène ou de soufre.

2. Composés selon la revendication 1 caractérisés en ce que X représente un atome d'oxygène.

3. Composés selon la revendication 1 qui répondent à l'une ou à l'autre des formules générales:

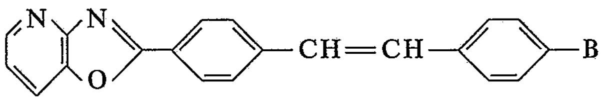

et

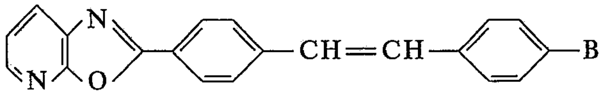

dans lesquelles B à la signification donnée à la revendication 1, $R^1$ représente un radical phényle éventuellement porteur d'un ou deux atomes de chlore ou d'un ou deux radicaux alkyles en $C_1-C_4$ ou $C_1-C_4$-alcoxy-$C_1-C_4$-alkyles, $R^2$, $R^3$, $R^4$ et $R^5$ ont la même signification que $R^1$ et peuvent en outre représenter chacun un radical alkyle en $C_1-C_6$, chloralkyle en $C_1-C_6$, $C_1-C_4$-alcoxy-$C_1-C_4$-alkyle, hydroxyalkyle en $C_1-C_4$, benzyle ou un radical $-(CH_2CH_2O)_n-R'$ dans lequel n est égal à 1 ou à 2 et R' désigne l'hydrogène ou un alkyle en $C_1-C_4$, et $R^3$, $R^4$ et $R^5$ peuvent aussi représenter chacun un atome d'hydrogène.

4. Composés selon la revendication 1 qui répondent à l'une des formules générales:

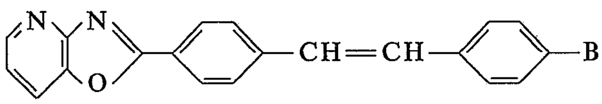

et

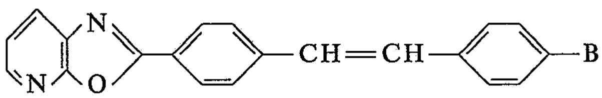

dans lesquelles B à la signification donnée à la revendication 1, $R^1$ représente un radical phényle, mono- ou di-$C_1-C_2$-alkylphényle, $C_1-C_2$-alkoxyphényle, chlorophényle ou dichlorophényle, $R^2$, $R^3$, $R^4$ et $R^5$ ont la même signification que $R^1$ et peuvent en outre représenter chacun un radical alkyle en $C_1-C_5$, un radical chloralkyle en $C_1-C_2$, un radical hydroxyalkyle en $C_1-C_2$, un radical $C_1-C_2$-alcoxy-$C_1-C_2$-alkyle ou un radical benzyle, et $R^3$, $R^4$ et $R^5$ peuvent aussi représenter chacun un atome d'hydrogène.

5. Procédé de préparation de composés selon l'une quelconque des revendications 1 à 4, procédé caractérisé en ce que:

a) lorsque B représente un groupe $-COOR^3$ ou

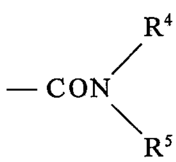

on fait réagir un acide carboxylique de formule générale:

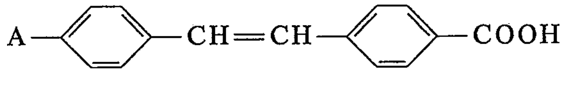

ou un dérivé fonctionnnel d'un tel acide, avec un composé de formule générale:

HOR$^3$ oder 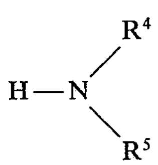

ou

b) lorsque B représente un groupe cyano on élimine de l'eau d'un composé de formule générale:

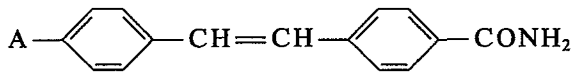

ou

c) lorsque B représente un radical de formule:

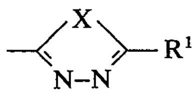

on fait réagir soit un acide de formule:

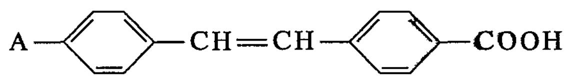

ou un dérivé fonctionnel d'un tel acide, avec un carbohydrazide de formule:

$H_2NNHCOR^1$

soit un acide de formule:

$R^1—COOH$

ou un dérivé fonctionnel d'un tel acide, avec un composé de formule:

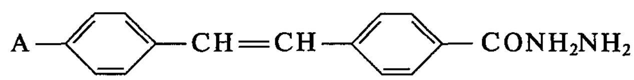

puis on cyclise avec un agent déshydratant, la cyclisation étant effectuée au moyen de $P_2S_5$ dans le cas où X est un atome de soufre,

ou

d) lorsque B représente un radical de formule:

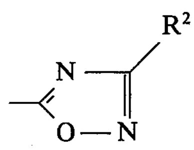

on fait réagir un acide de formule:

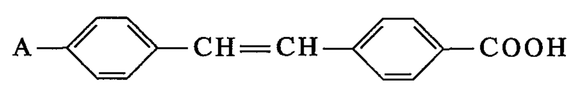

ou un dérivé fonctionnel d'un tel acide, avec une amideoxime de formule:

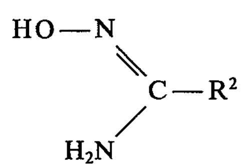

et on cyclise avec un agent déshydratant,

ou

e) lorsque B représente un radical de formule:

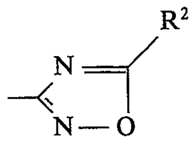

on fait réagir un composé de formule:

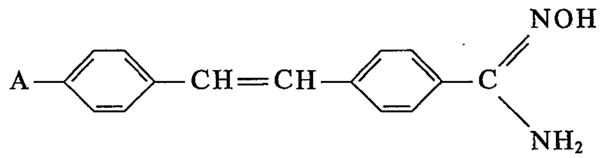

avec un chlorure d'acide carboxylique de formule:

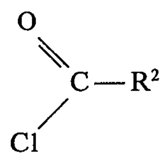

et on cyclise au moyen d'un agent déshydratant,
ou

f) on fait réagir un composé de formule:

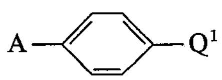

avec un composé de formule:

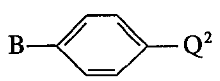

l'un des symboles $Q^1$ et $Q^2$ représentant un groupe formyle et l'autre un radical répondant à une des formules suivantes:

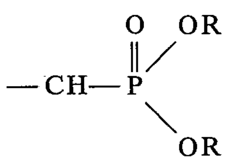

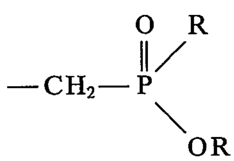

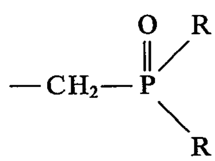

et

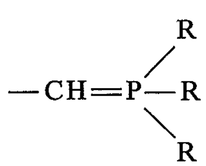

dans lesquelles R représente un radical alkyle éventuellement substitué, qui contient de préférence de l à 6 atomes de carbone, un radical aryle, de préférence phényle, un radical cycloalkyle ou un radical aralkyle, de préférence benzyle.

**6. Application des composés selon l'une quelconque des revendications 1 à 4 comme azureurs optiques.**